# EUROPEAN PATENT APPLICATION

(11) **EP 3 431 992 A1**
(43) Date of publication of application: **23.01.2019**
(21) Application number: 17766767.2
(22) Date of filing: 16.03.2017
(51) Int. Cl.: G01N 33/53, C12M 1/00, C12N 15/09, C12Q 1/06, C12Q 1/68

(54) **PD MARKER OF HEPATOCYTE GROWTH FACTOR (HGF)**

(30) Priority: 18.03.2016 JP 2016055491
(71) Applicant: Eisai R&D Management Co., Ltd., Tokyo 112-8088 (JP)
(72) Inventor: KIMURA, Mai, Tsukuba-shi Ibaraki 300-2635 (JP); MOTOI, Sotaro, Tsukuba-shi Ibaraki 300-2635 (JP); OBARA, Takashi, Tsukuba-shi Ibaraki 300-2635 (JP); MORIYA, Katsuhiro, Kobe-shi Hyogo 650-0047 (JP); OGASAWARA, Hideaki, Kobe-shi Hyogo 650-0047 (JP); ARITA, Yoshihisa, Kobe-shi Hyogo 650-0047 (JP); KAWANO, Tetsu, Kobe-shi Hyogo 650-0047 (JP)
(74) Representative: Hoffmann Eitle
(86) International application number: PCT/JP2017/010587
(87) International publication number: WO 2017/159771

(57) **Abstract**

The present invention provides a method for evaluating the pharmacological action of HGF that utilizes a PD marker in which fluctuation is recognized by administration of hepatocyte growth factor (HGF) in a broader range of subjects. A method for evaluating the pharmacodynamic action of HGF is provided. The aforementioned evaluation method is characterized in that it comprises a step of measuring the ApoA4 level in a biological sample collected from a test subject who was administered HGF. According to the present invention, a kit for evaluating the pharmacodynamic action of HGF comprising an anti-ApoA4 antibody is further provided.

SEQUENCE LISTING

<110>  EISAI R&D MANAGEMENT CO., LTD.

<120>  PD marker for a hepatocyte growth factor (HGF)

<130>  ESAP1602F

<150>  JP2016-055491
<151>  2016-03-18

<160>  5

<170>  PatentIn version 3.5

<210>  1
<211>  697
<212>  PRT
<213>  Homo sapiens

<400>  1

Gln Arg Lys Arg Arg Asn Thr Ile His Glu Phe Lys Lys Ser Ala Lys
1               5                   10                  15

Thr Thr Leu Ile Lys Ile Asp Pro Ala Leu Lys Ile Lys Thr Lys Lys
            20                  25                  30

Val Asn Thr Ala Asp Gln Cys Ala Asn Arg Cys Thr Arg Asn Lys Gly
        35                  40                  45

Leu Pro Phe Thr Cys Lys Ala Phe Val Phe Asp Lys Ala Arg Lys Gln
    50                  55                  60

Cys Leu Trp Phe Pro Phe Asn Ser Met Ser Ser Gly Val Lys Lys Glu
65                  70                  75                  80

Phe Gly His Glu Phe Asp Leu Tyr Glu Asn Lys Asp Tyr Ile Arg Asn
                85                  90                  95

Cys Ile Ile Gly Lys Gly Arg Ser Tyr Lys Gly Thr Val Ser Ile Thr
            100                 105                 110

Lys Ser Gly Ile Lys Cys Gln Pro Trp Ser Ser Met Ile Pro His Glu
        115                 120                 125

His Ser Phe Leu Pro Ser Ser Tyr Arg Gly Lys Asp Leu Gln Glu Asn
    130                 135                 140

Tyr Cys Arg Asn Pro Arg Gly Glu Glu Gly Gly Pro Trp Cys Phe Thr
145                 150                 155                 160

Ser Asn Pro Glu Val Arg Tyr Glu Val Cys Asp Ile Pro Gln Cys Ser

165                 170                 175

Glu Val Glu Cys Met Thr Cys Asn Gly Glu Ser Tyr Arg Gly Leu Met
            180                 185                 190

Asp His Thr Glu Ser Gly Lys Ile Cys Gln Arg Trp Asp His Gln Thr
        195                 200                 205

Pro His Arg His Lys Phe Leu Pro Glu Arg Tyr Pro Asp Lys Gly Phe
    210                 215                 220

Asp Asp Asn Tyr Cys Arg Asn Pro Asp Gly Gln Pro Arg Pro Trp Cys
225                 230                 235                 240

Tyr Thr Leu Asp Pro His Thr Arg Trp Glu Tyr Cys Ala Ile Lys Thr
                245                 250                 255

Cys Ala Asp Asn Thr Met Asn Asp Thr Asp Val Pro Leu Glu Thr Thr
            260                 265                 270

Glu Cys Ile Gln Gly Gln Gly Glu Gly Tyr Arg Gly Thr Val Asn Thr
        275                 280                 285

Ile Trp Asn Gly Ile Pro Cys Gln Arg Trp Asp Ser Gln Tyr Pro His
    290                 295                 300

Glu His Asp Met Thr Pro Glu Asn Phe Lys Cys Lys Asp Leu Arg Glu
305                 310                 315                 320

Asn Tyr Cys Arg Asn Pro Asp Gly Ser Glu Ser Pro Trp Cys Phe Thr
                325                 330                 335

Thr Asp Pro Asn Ile Arg Val Gly Tyr Cys Ser Gln Ile Pro Asn Cys
            340                 345                 350

Asp Met Ser His Gly Gln Asp Cys Tyr Arg Gly Asn Gly Lys Asn Tyr
        355                 360                 365

Met Gly Asn Leu Ser Gln Thr Arg Ser Gly Leu Thr Cys Ser Met Trp
    370                 375                 380

Asp Lys Asn Met Glu Asp Leu His Arg His Ile Phe Trp Glu Pro Asp
385                 390                 395                 400

Ala Ser Lys Leu Asn Glu Asn Tyr Cys Arg Asn Pro Asp Asp Asp Ala
                405                 410                 415

His Gly Pro Trp Cys Tyr Thr Gly Asn Pro Leu Ile Pro Trp Asp Tyr
            420                 425                 430

Cys Pro Ile Ser Arg Cys Glu Gly Asp Thr Thr Pro Thr Ile Val Asn
        435                 440                 445

Leu Asp His Pro Val Ile Ser Cys Ala Lys Thr Lys Gln Leu Arg Val
    450                 455                 460

Val Asn Gly Ile Pro Thr Arg Thr Asn Ile Gly Trp Met Val Ser Leu
465                 470                 475                 480

Arg Tyr Arg Asn Lys His Ile Cys Gly Gly Ser Leu Ile Lys Glu Ser
                485                 490                 495

Trp Val Leu Thr Ala Arg Gln Cys Phe Pro Ser Arg Asp Leu Lys Asp
            500                 505                 510

Tyr Glu Ala Trp Leu Gly Ile His Asp Val His Gly Arg Gly Asp Glu
        515                 520                 525

Lys Cys Lys Gln Val Leu Asn Val Ser Gln Leu Val Tyr Gly Pro Glu
    530                 535                 540

Gly Ser Asp Leu Val Leu Met Lys Leu Ala Arg Pro Ala Val Leu Asp
545                 550                 555                 560

Asp Phe Val Ser Thr Ile Asp Leu Pro Asn Tyr Gly Cys Thr Ile Pro
                565                 570                 575

Glu Lys Thr Ser Cys Ser Val Tyr Gly Trp Gly Tyr Thr Gly Leu Ile
            580                 585                 590

Asn Tyr Asp Gly Leu Leu Arg Val Ala His Leu Tyr Ile Met Gly Asn
        595                 600                 605

Glu Lys Cys Ser Gln His His Arg Gly Lys Val Thr Leu Asn Glu Ser
    610                 615                 620

Glu Ile Cys Ala Gly Ala Glu Lys Ile Gly Ser Gly Pro Cys Glu Gly
625                 630                 635                 640

Asp Tyr Gly Gly Pro Leu Val Cys Glu Gln His Lys Met Arg Met Val
                645                 650                 655

Leu Gly Val Ile Val Pro Gly Arg Gly Cys Ala Ile Pro Asn Arg Pro
            660                 665                 670

Gly Ile Phe Val Arg Val Ala Tyr Tyr Ala Lys Trp Ile His Lys Ile
        675                 680                 685

Ile Leu Thr Tyr Lys Val Pro Gln Ser
    690                 695

<210>  2
<211>  1460
<212>  DNA
<213>  Homo sapiens

<400>  2
tgcagcgcag gtgagctctc ctgaggacct ctctgtcagc tcccctgatt gtagggagga      60

tccagtgtgg caagaaactc ctccagccca gcaagcagct caggatgttc ctgaaggccg     120

tggtcctgac cctggccctg gtggctgtcg ccggagccag ggctgaggtc agtgctgacc     180

aggtggccac ggtgatgtgg gactacttca gccagctgag caacaatgcc aaggaggccg     240

tggaacatct ccagaaatct gaactcaccc agcaactcaa tgccctcttc caggacaaac     300

ttggagaagt gaacacttac gcaggtgacc tgcagaagaa gctggtgccc tttgccaccg     360

agctgcatga acgcctggcc aaggactcgg agaaactgaa ggaggagatt gggaaggagc     420

tggaggagct gagggcccgg ctgctgcccc atgccaatga ggtgagccag aagatcgggg     480

acaacctgcg agagcttcag cagcgcctgg agccctacgc ggaccagctg cgcacccagg     540

tcagcacgca ggccgagcag ctgcggcgcc agctgacccc ctacgcacag cgcatggaga     600

gagtgctgcg ggagaacgcc gacagcctgc aggcctcgct gaggccccac gccgacgagc     660

tcaaggccaa gatcgaccag aacgtggagg agctcaaggg acgccttacg ccctacgctg     720

acgaattcaa agtcaagatt gaccagaccg tggaggagct gcgccgcagc ctggctccct     780

atgctcagga cacgcaggag aagctcaacc accagcttga gggcctgacc ttccagatga     840

agaagaacgc cgaggagctc aaggccagga tctcggccag tgccgaggag ctgcggcaga     900

ggctggcgcc cttggccgag gacgtgcgtg gcaacctgag gggcaacacc gaggggctgc     960

agaagtcact ggcagagctg ggtgggcacc tggaccagca ggtggaggag ttccgacgcc    1020

gggtggagcc ctacggggaa aacttcaaca aagccctggt gcagcagatg gaacagctca    1080

ggcagaaact gggcccccat gcgggggacg tggaaggcca cttgagcttc ctggagaagg    1140

acctgaggga caaggtcaac tccttcttca gcaccttcaa ggagaaagag agccaggaca    1200

agactctctc cctccctgag ctggagcaac agcaggaaca gcagcaggag cagcagcagg    1260

agcaggtgca gatgctggcc cctttggaga gctgagctgc ccctggtgca ctggccccac    1320

cctcgtggac acctgccctg ccctgccacc tgtctgtctg tctgtcccaa agaagttctg    1380

gtatgaactt gaggacacat gtccagtggg aggtgagacc acctctcaat attcaataaa    1440

gctgctgaga atctagcctc                                                1460

<210>  3
<211>  376
<212>  PRT
<213>  Homo sapiens

<400>  3

Glu Val Ser Ala Asp Gln Val Ala Thr Val Met Trp Asp Tyr Phe Ser
1               5                   10                  15

Gln Leu Ser Asn Asn Ala Lys Glu Ala Val Glu His Leu Gln Lys Ser
            20                  25                  30

Glu Leu Thr Gln Gln Leu Asn Ala Leu Phe Gln Asp Lys Leu Gly Glu
        35                  40                  45

Val Asn Thr Tyr Ala Gly Asp Leu Gln Lys Lys Leu Val Pro Phe Ala
    50                  55                  60

Thr Glu Leu His Glu Arg Leu Ala Lys Asp Ser Glu Lys Leu Lys Glu
65                  70                  75                  80

Glu Ile Gly Lys Glu Leu Glu Glu Leu Arg Ala Arg Leu Leu Pro His
                85                  90                  95

Ala Asn Glu Val Ser Gln Lys Ile Gly Asp Asn Leu Arg Glu Leu Gln
            100                 105                 110

Gln Arg Leu Glu Pro Tyr Ala Asp Gln Leu Arg Thr Gln Val Ser Thr
        115                 120                 125

Gln Ala Glu Gln Leu Arg Arg Gln Leu Thr Pro Tyr Ala Gln Arg Met
    130                 135                 140

Glu Arg Val Leu Arg Glu Asn Ala Asp Ser Leu Gln Ala Ser Leu Arg
145                 150                 155                 160

Pro His Ala Asp Glu Leu Lys Ala Lys Ile Asp Gln Asn Val Glu Glu
                165                 170                 175

Leu Lys Gly Arg Leu Thr Pro Tyr Ala Asp Glu Phe Lys Val Lys Ile
            180                 185                 190

Asp Gln Thr Val Glu Glu Leu Arg Arg Ser Leu Ala Pro Tyr Ala Gln
        195                 200                 205

Asp Thr Gln Glu Lys Leu Asn His Gln Leu Glu Gly Leu Thr Phe Gln

210                 215                 220

Met Lys Lys Asn Ala Glu Glu Leu Lys Ala Arg Ile Ser Ala Ser Ala
225                 230                 235                 240

Glu Glu Leu Arg Gln Arg Leu Ala Pro Leu Ala Glu Asp Val Arg Gly
                245                 250                 255

Asn Leu Arg Gly Asn Thr Glu Gly Leu Gln Lys Ser Leu Ala Glu Leu
            260                 265                 270

Gly Gly His Leu Asp Gln Gln Val Glu Glu Phe Arg Arg Arg Val Glu
        275                 280                 285

Pro Tyr Gly Glu Asn Phe Asn Lys Ala Leu Val Gln Gln Met Glu Gln
    290                 295                 300

Leu Arg Gln Lys Leu Gly Pro His Ala Gly Asp Val Glu Gly His Leu
305                 310                 315                 320

Ser Phe Leu Glu Lys Asp Leu Arg Asp Lys Val Asn Ser Phe Phe Ser
                325                 330                 335

Thr Phe Lys Glu Lys Glu Ser Gln Asp Lys Thr Leu Ser Leu Pro Glu
            340                 345                 350

Leu Glu Gln Gln Gln Glu Gln Gln Gln Glu Gln Gln Gln Glu Gln Val
        355                 360                 365

Gln Met Leu Ala Pro Leu Glu Ser
    370                 375

<210>  4
<211>  1460
<212>  DNA
<213>  Homo sapiens

<400>  4
tgcagcgcag gtgagctctc ctgaggacct ctctgtcagc tcccctgatt gtagggagga      60

tccagtgtgg caagaaactc ctccagccca gcaagcagct caggatgttc ctgaaggccg     120

tggtcctgac cctggccctg gtggctgtcg ccggagccag ggctgaggtc agtgctgacc     180

aggtggccac ggtgatgtgg gactacttca gccagctgag caacaatgcc aaggaggccg     240

tggaacatct ccagaaatct gaactcaccc agcaactcaa tgccctcttc caggacaaac     300

ttggagaagt gaacacttac gcaggtgacc tgcagaagaa gctggtgccc tttgccaccg     360

agctgcatga acgcctggcc aaggactcgg agaaactgaa ggaggagatt gggaaggagc     420

tggaggagct gagggcccgg ctgctgcccc atgccaatga ggtgagccag aagatcgggg 480

acaacctgcg agagcttcag cagcgcctgg agccctacgc ggaccagctg cgcacccagg 540

tcaccacgca ggccgagcag ctgcggcgcc agctgacccc ctacgcacag cgcatggaga 600

gagtgctgcg ggagaacgcc gacagcctgc aggcctcgct gaggccccac gccgacgagc 660

tcaaggccaa gatcgaccag aacgtggagg agctcaaggg acgccttacg ccctacgctg 720

acgaattcaa agtcaagatt gaccagaccg tggaggagct gcgccgcagc ctggctccct 780

atgctcagga cacgcaggag aagctcaacc accagcttga gggcctgacc ttccagatga 840

agaagaacgc cgaggagctc aaggccagga tctcggccag tgccgaggag ctgcggcaga 900

ggctggcgcc cttggccgag gacgtgcgtg gcaacctgag gggcaacacc gaggggctgc 960

agaagtcact ggcagagctg ggtgggcacc tggaccagca ggtggaggag ttccgacgcc 1020

gggtggagcc ctacggggaa aacttcaaca aagccctggt gcagcagatg gaacagctca 1080

ggcagaaact gggcccccat gcgggggacg tggaaggcca cttgagcttc ctggagaagg 1140

acctgaggga caaggtcaac tccttcttca gcaccttcaa ggagaaagag agccaggaca 1200

agactctctc cctccctgag ctggagcaac agcaggaaca gcagcaggag cagcagcagg 1260

agcaggtgca gatgctggcc cctttggaga gctgagctgc ccctggtgca ctggccccac 1320

cctcgtggac acctgccctg ccctgccacc tgtctgtctg tctgtcccaa agaagttctg 1380

gtatgaactt gaggacacat gtccagtggg aggtgagacc acctctcaat attcaataaa 1440

gctgctgaga atctagcctc 1460

<210> 5
<211> 376
<212> PRT
<213> Homo sapiens

<400> 5

Glu Val Ser Ala Asp Gln Val Ala Thr Val Met Trp Asp Tyr Phe Ser
1               5                   10                  15

Gln Leu Ser Asn Asn Ala Lys Glu Ala Val Glu His Leu Gln Lys Ser
            20                  25                  30

Glu Leu Thr Gln Gln Leu Asn Ala Leu Phe Gln Asp Lys Leu Gly Glu
        35                  40                  45

Val Asn Thr Tyr Ala Gly Asp Leu Gln Lys Lys Leu Val Pro Phe Ala
    50                  55                  60

Thr Glu Leu His Glu Arg Leu Ala Lys Asp Ser Glu Lys Leu Lys Glu
65                  70                  75                  80

Glu Ile Gly Lys Glu Leu Glu Glu Leu Arg Ala Arg Leu Leu Pro His
                85                  90                  95

Ala Asn Glu Val Ser Gln Lys Ile Gly Asp Asn Leu Arg Glu Leu Gln
            100                 105                 110

Gln Arg Leu Glu Pro Tyr Ala Asp Gln Leu Arg Thr Gln Val Asn Thr
        115                 120                 125

Gln Ala Glu Gln Leu Arg Arg Gln Leu Thr Pro Tyr Ala Gln Arg Met
    130                 135                 140

Glu Arg Val Leu Arg Glu Asn Ala Asp Ser Leu Gln Ala Ser Leu Arg
145                 150                 155                 160

Pro His Ala Asp Glu Leu Lys Ala Lys Ile Asp Gln Asn Val Glu Glu
                165                 170                 175

Leu Lys Gly Arg Leu Thr Pro Tyr Ala Asp Glu Phe Lys Val Lys Ile
            180                 185                 190

Asp Gln Thr Val Glu Glu Leu Arg Arg Ser Leu Ala Pro Tyr Ala Gln
        195                 200                 205

Asp Thr Gln Glu Lys Leu Asn His Gln Leu Glu Gly Leu Thr Phe Gln
    210                 215                 220

Met Lys Lys Asn Ala Glu Glu Leu Lys Ala Arg Ile Ser Ala Ser Ala
225                 230                 235                 240

Glu Glu Leu Arg Gln Arg Leu Ala Pro Leu Ala Glu Asp Val Arg Gly
                245                 250                 255

Asn Leu Arg Gly Asn Thr Glu Gly Leu Gln Lys Ser Leu Ala Glu Leu
            260                 265                 270

Gly Gly His Leu Asp Gln Gln Val Glu Glu Phe Arg Arg Arg Val Glu
        275                 280                 285

Pro Tyr Gly Glu Asn Phe Asn Lys Ala Leu Val Gln Gln Met Glu Gln
    290                 295                 300

Leu Arg Gln Lys Leu Gly Pro His Ala Gly Asp Val Glu Gly His Leu
305                 310                 315                 320

Ser Phe Leu Glu Lys Asp Leu Arg Asp Lys Val Asn Ser Phe Phe Ser
                325                 330                 335

Thr Phe Lys Glu Lys Glu Ser Gln Asp Lys Thr Leu Ser Leu Pro Glu
            340                 345                 350

Leu Glu Gln Gln Gln Glu Gln Gln Gln Glu Gln Gln Gln Glu Gln Val
        355                 360                 365

Gln Met Leu Ala Pro Leu Glu Ser
    370                 375

## Description

### Technical Field

The present invention relates to a PD marker of hepatocyte growth factor (hereinafter also referred to as HGF) and utilization thereof.

### Background Art

HGF is a factor having hepatic parenchymal cell proliferation activity that is purified from the blood plasma of human fulminant hepatitis patients (Patent Literature 1 and Non-Patent Literature 1), and is reported as having various pharmacological effects such as antitumoral effect, enhancement of cell-mediated immunity, wound therapeutic effect, and tissue regeneration promotional effect (such as Patent Literature 2).

Development of a biomarker (PD marker) that reflects pharmacodynamic action is important in order to increase the likelihood of drug development, and for HGF, α-fetoprotein and soluble Fas have been previously proposed as indicators for monitoring the drug effect of HGF against patients with liver disorder (Patent Literature 3).

However, since all of these markers are blood markers that reflect pathological improvement of liver disease, they do not show that the administered HGF shows pharmacodynamic action.

### Citation List

### Patent Literatures

[Patent Literature 1] Japanese Published Unexamined Patent Application Publication No. S63-22526
[Patent Literature 2] Japanese Patent No. 2747979
[Patent Literature 3] International Publication WO2012/144535

### Non-Patent Literatures

[Non-Patent Literature 1] J. Clin. Invest., 81, 414(1988)

### Summary of the Invention

### Problems to be Solved by the Invention

The object of the present invention is to provide a method for evaluating the pharmacological action of HGF that utilizes a PD marker in which fluctuation is recognized by administration of HGF in a broader range of subjects.

### Means for Solving the Problems

In the process of repeated extensive investigation by the present inventors to solve the above problem, the present inventors found apolipoprotein A-IV (ApoA4) as one of the markers where expression is fluctuated by administration of HGF. Upon progressing further research on ApoA4, the present inventors found that expression of ApoA4 gene is induced by interaction between HGF and c-Met, induction of ApoA4 gene expression can be detected with ApoA4 protein in blood, and increase in ApoA4 gene expression by exposure to HGF is a recognized marker also in normal human hepatocytes.

The present invention is based on the above knowledge, and encompasses the following aspects.
[1] A method for evaluating the pharmacodynamic action of hepatocyte growth factor (HGF), characterized in that it comprises a step of measuring the ApoA4 level in a biological sample collected from a test subject who was administered HGF.
[2] The evaluation method according to [1], characterized in that it further comprises a step of comparing the ApoA4 level in a biological sample collected from a test subject who was administered HGF with the ApoA4 level in said biological sample collected from a test subject who was not administered HGF or who has a disappeared influence from the administration of HGF.
[3] The evaluation method according to [2], characterized in that it further comprises a step of determining that said administered HGF is administered in an amount that yields pharmacodynamic action when the ApoA4 level of a test subject who was administered HGF is elevated compared to the ApoA4 level of a test subject who was not administered HGF or who has a disappeared influence from the administration of HGF.
[4] The evaluation method according to any of [1] to [3], characterized in that said "ApoA4 level" is the expression level of ApoA4 protein.
[5] The evaluation method according to [4], characterized in that the expression level of ApoA4 protein in the biologically derived sample is measured by an immunoassay.
[6] The evaluation method according to any of [1] to [5], characterized in that said biologically derived sample is a sample derived from serum or blood plasma.
[7] The evaluation method according to any of [1] to [3], characterized in that said "ApoA4 level" is the expression level of ApoA4 gene.
[8] The evaluation method according to [7], characterized in that the expression level of ApoA4 gene in the biologically derived sample is measured by real-time PCR.
[9] The evaluation method according to any of [1] to [3], [7], or [8], characterized in that said biological sample is a hepatocyte or hepatic tissue.
[10] The evaluation method according to any of [1] to [9], characterized in that said test subject is a healthy individual.
[11] The evaluation method according to any of [1] to [9], characterized in that said test subject is a test subject having liver disease.
[12] A kit for evaluating the pharmacodynamic action of HGF, characterized in that it comprises an anti-ApoA4 antibody.

Those skilled in the art shall recognize that an invention of any combination of one or more characteristics of the present invention described above is also encompassed by the scope of the present invention.

### Effects of the Invention

According to the present invention, a method for evaluating the pharmacodynamic action of HGF that utilizes ApoA4 as a PD marker in which fluctuation is recognized by administration of HGF in a broader range of subjects is provided.

### Brief Description of the Drawings

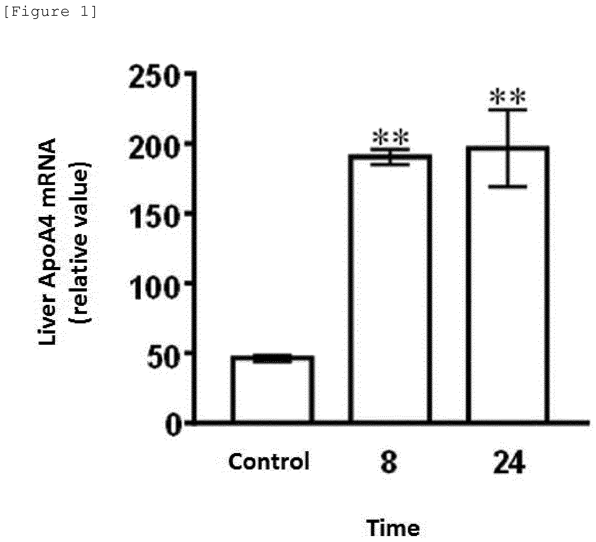

Figure 1 shows the result of real-time PCR on ApoA4 mRNA extracted from the liver of mice that were administered recombinant human-HGF (rh-HGF). **P<0.01: Comparison between the control group and the rh-HGF treatment group (Dunnett's multiple comparison test).

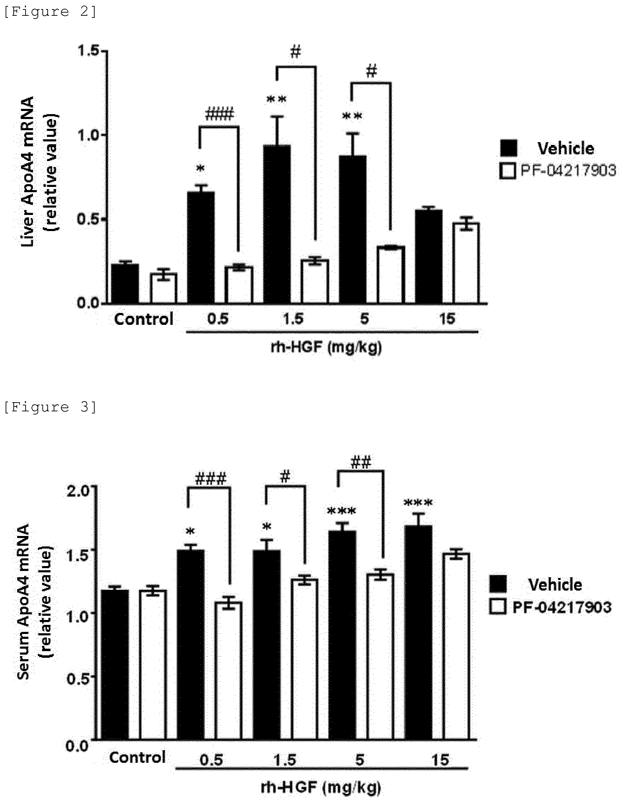

Figure 2 shows the result of real-time PCR on ApoA4 mRNA extracted from the liver of mice that were administered rh-HGF and c-Met inhibitor. *P<0.05, **P<0.01: Comparison between the control group and the rh-HGF treatment group (Dunnett's multiple comparison test). #P<0.05, ###P<0.001: Comparison between the vehicle treatment group and the PF-04217903 treatment group (unpaired t-test).
Figure 3 shows the quantification result of ApoA4 protein in the serum derived from mice that were administered rh-HGF, or rh-HGF and c-Met inhibitor. *P<0.05, **P<0.001: Comparison between the control group and the rh-HGF treatment group (Dunnett's multiple comparison test). #P<0.05, ##P<0.01, ###P<0.001: Comparison between the vehicle treatment group and the PF-04217903 treatment group (unpaired t-test).

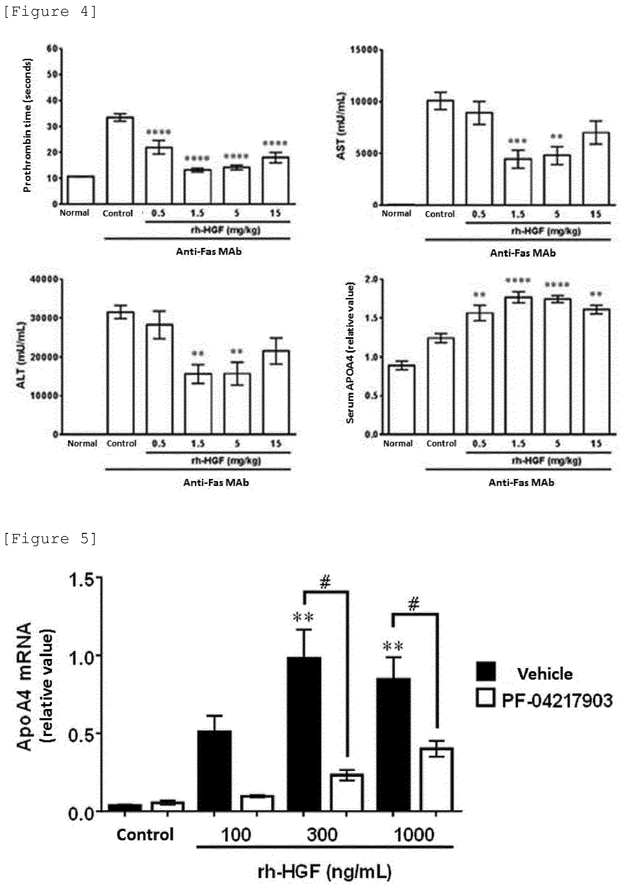

Figure 4 shows the quantification result of ApoA4 protein in the serum derived from Jo2 liver failure mice that were administered rh-HGF. *P<0.01, ***P<0.0001: Comparison between the control group and the rh-HGF treatment group (Dunnett's multiple comparison test). Anti-Fas MAb: Anti-Fas/CD95 monoclonal antibody.
Figure 5 shows the result of real-time PCR on ApoA4 mRNA in samples obtained from human hepatocyte cultures treated with rh-HGF. **P<0.01: Comparison between the control group and the rh-HGF treatment group (Dunnett's multiple comparison test). #P<0.05: Comparison between the vehicle treatment group and the PF-04217903 treatment group (unpaired t-test).

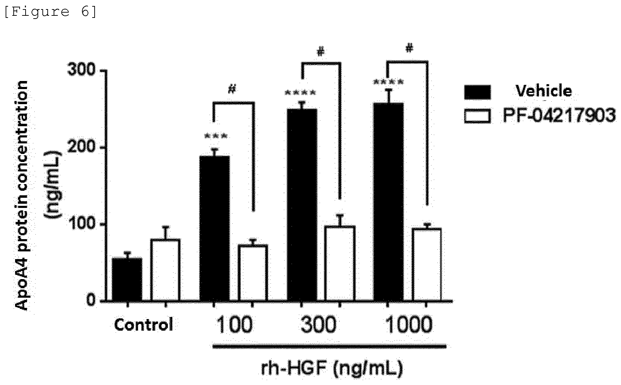

Figure 6 shows the result of quantifying ApoA4 protein in human hepatocyte culture supernatants treated with rh-HGF by Western blot method. ***P<0.001, ****P<0.0001: Comparison between the control group and the rh-HGF treatment group (Dunnett's multiple comparison test). #P<0.05: Comparison between the vehicle treatment group and the PF-04217903 treatment group (unpaired t-test).

### Description of Embodiments

The embodiments of the present invention will now be specifically described below.

The present invention relates to a method for evaluating the pharmacodynamic action of HGF. The evaluation method of the present invention is based on a new finding that the ApoA4 level in a subject is fluctuated by administration of HGF. Accordingly, the evaluation method of the present invention is characterized in that it comprises a step of measuring the ApoA4 level in a biological sample collected from a test subject who was administered HGF.

HGF used in the present invention may be that commercially available or manufactured by recombinant technology. Manufacture of HGF by recombinant technology can be done for example by cloning the gene encoding HGF, creating a vector comprising the aforementioned gene, introducing this into a host cell to allow transformation to thereby obtain a cell expressing HGF, and subjecting this to cell culture. The cell, the type of vector, the type of cell, culturing condition, and the like used for this process are within the technical range of those skilled in the art, and they can set appropriate conditions as required.

In the present invention, the test subject to be the target for evaluation is not particularly limited, and includes for example a test subject who has developed liver disease that is to be the target of HGF administration, a test subject who has the risk of developing liver disease, and a healthy individual. In the present invention, liver disease may be any liver disease, but is preferably severe hepatopathy. A test subject typically refers to a human, but may also be for example other primates, rodents, and the like as long as it is an animal in which ApoA4 expression is recognized.

The administration route of HGF is not particularly limited, but in light of the fact that HGF is a protein product, it is typically by intravenous administration.

In the present invention, said "biological sample collected from a test subject" is not particularly limited as long as it can be collected from a test subject, and can be for example tissues (formed by cells, the basic units configuring animals, that have differentiated and formed clusters so as to exert particular functions, is configured by cells and intercellular substances, and achieves a certain function as a whole), organs (created by orderly combination of tissues), or body fluids. Tissues or organs include, nonlimitingly, ovary, uterus, breast, thyroid, brain, esophagus, tongue, lung, pancreas, stomach, small intestine, duodenum, large intestine, bladder, kidney, liver, prostate, gallbladder, pharynx, muscle, bone, skin, and the like, but are not limited thereto. Body fluids include, nonlimitingly, blood such as serum, blood plasma or whole blood, lymph, tissue fluid, spinal fluid, body cavity fluid, digestive juice, nasal discharge, lacrimal fluid, sweat, urine, and the like, but are not limited thereto. In terms of ease of acquirement and treatment, it is preferred to employ serum or blood plasma as said biological sample. Moreover, said body fluid may be a body fluid as it is collected from a subject, or those subjected to treatments ordinarily performed on collected body fluids such as dilution and concentration. Note that the person who collects or prepares the biological sample derived from a test subject employed in the present invention may be the same or different individual than who carries out the steps of the present invention. Moreover, the biological sample derived from a test subject employed in the present invention may be collected or prepared upon carrying out the present invention, or may be collected or prepared and stored in advance.

In one embodiment, the present invention further comprises a step of comparing the ApoA4 level in a biological sample collected from a test subject who was administered HGF with the ApoA4 level in said biological sample collected from a test subject who was not administered HGF or who has a disappeared influence from the administration of HGF. "A test subject who has a disappeared influence from the administration of HGF " refers to a test subject who has a history of HGF administration but a length of time sufficient for the pharmacodynamic action by HGF to disappear has passed.

In a further preferred embodiment, the present invention further comprises a step of determining that said administered HGF is administered in an amount that yields pharmacological action when the ApoA4 level of a test subject who was administered HGF is elevated compared to the ApoA4 level of a test subject who was not administered HGF or who has a disappeared influence from the administration of HGF.

In the present invention, "administered in an amount that yields pharmacodynamic action" means that some kind of pharmacodynamic action is provided by administration of HGF, and the magnitude of the action is not necessarily an issue. For example, both of when the ApoA4 level had largely elevated or when the ApoA4 level had only slightly elevated after administration of HGF falls under "administered in an amount that yields pharmacodynamic action." In this regard, "administered in an amount that yields pharmacodynamic action" is not synonymous with "administered in a therapeutically effective amount," and depending on the degree of elevation of the ApoA4 level, may be determined to be "administered in a therapeutically effective amount" or may be determined not to be "administered in a therapeutically effective amount."

In the present invention, HGF may include HGF derived from human, mouse, rat, pig, and other animals, but is preferably HGF derived from human.

In the present invention, human HGF (hHGF) includes a polypeptide having the amino acid sequence shown in SEQ ID NO. 1 or a variant thereof. Variants of human HGF include a polypeptide having an amino acid sequence having addition, deletion, or substitution of one or multiple amino acids to the amino acid sequence shown in SEQ ID NO. 1, as well as having HGF activity similar to or more than the polypeptide having the amino acid sequence shown in SEQ ID NO. 1. "Multiple" as used herein is 2 - 150, more preferably 2 - 80, more preferably 2 - 70, more preferably 2 - 60, more preferably 2 - 50, more preferably 2 - 40, more preferably 2 - 30, more preferably 2 - 20, more preferably 2 - 10, or more preferably 2 - 5. Said variants also include a polypeptide having an amino acid sequence showing at least 80%, more preferably at least 85%, and more preferably at least 90%, 91%, 92%, 93%, 94%, 95%, 96%, 97%, 98%, or 99% sequence identity with the amino acid sequence shown in SEQ ID NO. 1, as well as having HGF activity similar to or more than the polypeptide having the amino acid sequence shown in SEQ ID NO. 1, or a polypeptide having an amino acid sequence encoded by a gene that hybridizes under stringent conditions to the gene encoding the amino acid sequence shown in SEQ ID NO. 1, as well as having HGF activity similar to or more than the polypeptide having the amino acid sequence shown in SEQ ID NO. 1.

In the present invention, a "stringent condition" can include those where in the post hybridization washing, hybridization is achieved with washing at for example a condition of "2 X SSC, 0.1% SDS, 50°C," a condition of "2 X SSC, 0.1% SDS, 42°C, " or a condition of "1 X SSC, 0.1% SDS, 37°C, " and a more stringent condition can include those where hybridization is achieved with washing at for example conditions of "2 X SSC, 0.1% SDS, 65°C," "0.5 X SSC, 0.1% SDS, 42°C," "0.2 X SSC, 0.1% SDS, 65°C," or "0.1 X SSC, 0.1% SDS, 65°C" (1 X SSC is 150 mM NaCl, 15 mM sodium citrate, pH 7.0). More particularly, as a method that employs Rapid-hyb buffer (Amersham Life Science), it is conceivable to perform prehybridization at 68°C for 30 minutes or more, after which a probe is added and retained at 68°C for 1 hour or more to allow formation of hybrids, and then to perform three washes in 2 X SSC and 0.1% SDS at room temperature for 20 minutes, three washes in 1 X SSC and 0.1% SDS at 37°C for 20 minutes, and finally two washes in 1 X SSC and 0.1% SDS at 50°C for 20 minutes. More preferably, for example a solution comprising 5 X SSC, 7% (W/V) SDS, 100 µg/ml denatured salmon sperm DNA, and 5 X Denhardt's solution (1 X Denhardt's solution comprises 0.2% polyvinylpyrrolidone, 0.2% bovine serum albumin, and 0.2% Ficoll) is employed as prehybridization and hybridization solutions, prehybridization is performed at 65°C for 30 minutes to 1 hour, and hybridization is performed at the same temperature overnight (6 - 8 hours). In addition, it is also possible to perform for example prehybridization in Expresshyb Hybridization Solution (CLONTECH) at 55°C for 30 minutes or more, add a labeled probe and incubate at 37 - 55°C for 1 hour or more, and three washes in 2 X SSC and 0.1% SDS at room temperature for 20 minutes and then one washing in 1 X SSC and 0.1% SDS at 37°C for 20 minutes. Here, a more stringent condition can be achieved for example by raising the temperature for prehybridization, hybridization, or second washing. For example, the temperature for prehybridization and hybridization can be 60°C, or 65°C or 68°C for a further stringent condition. Those skilled in the art will be able to set conditions for obtaining isoforms, allelic variants, and corresponding genes derived from other organism species for the gene of the present invention by factoring in various conditions such as other probe concentration, probe length, and reaction time in addition to conditions such as salt concentration of such a buffer and temperature. For a detailed protocol of the hybridization method, reference can be made to "Molecular Cloning, A Laboratory Manual 2nd ed." (Cold Spring Harbor Press (1989); in particular Section 9.47-9.58), "Current Protocols in Molecular Biology" (John Wiley & Sons (1987-1997); in particular Section 6.3-6.4), "DNA Cloning 1: Core Techniques, A Practical Approach 2nd ed." (Oxford University (1995); in particular Section 2.10 for conditions), and the like.

"ApoA4 gene" is the gene encoding apolipoprotein A-IV, and is known to be expressed in human as well as in rodents such as mouse, rat, and the like. The gene sequence of human ApoA4 and the amino acid sequence corresponding thereto are registered as GenBank Accession No. NM_000482 and GenBank Accession No. NP_000473, respectively.

In the present invention, human ApoA4 protein may comprise a variant thereof. Variants of human ApoA4 protein include a polypeptide having an amino acid sequence having addition, deletion, or substitution of one or multiple amino acids to the amino acid sequence shown in SEQ ID NO. 3, as well as having functional property equivalent to that of human ApoA4 protein having the amino acid sequence shown in SEQ ID NO. 3. "Multiple" as used herein is 2 - 80, more preferably 2 - 60, more preferably 2 - 40, more preferably 2 - 35, more preferably 2 - 30, more preferably 2 - 25, more preferably 2 - 20, more preferably 2 - 15, more preferably 2 - 10, or more preferably 2 - 5. Said variants also include a polypeptide having an amino acid sequence showing at least 80%, more preferably at least 85%, and more preferably at least 90%, 91%, 92%, 93%, 94%, 95%, 96%, 97%, 98%, or 99% sequence identity with the amino acid sequence shown in SEQ ID NO. 3, as well as having functional property equivalent to that of human ApoA4 protein having the amino acid sequence shown in SEQ ID NO. 3, or a polypeptide having an amino acid sequence encoded by a gene that hybridizes under stringent conditions to the gene encoding the amino acid sequence shown in SEQ ID NO. 3 (such as SEQ ID NO. 2), as well as having functional property equivalent to that of human ApoA4 protein having the amino acid sequence shown in SEQ ID NO. 3. In an exemplary embodiment, variants of human ApoA4 protein have the amino acid sequence shown in SEQ ID NO. 5, or an amino acid sequence encoded by the base sequence shown in SEQ ID NO. 4.

The measurement of the ApoA4 level in said biological sample is not particularly limited as long as it is a method that can quantitatively detect ApoA4. Accordingly, it may be by measuring the expression level of ApoA4 protein or by measuring the expression level of ApoA4 gene (i.e. ApoA4 mRNA). Since fluctuation of ApoA4 protein due to HGF administration can be confirmed by protein in the blood, measurement of the amount of ApoA4 protein in the serum or blood plasma may be advantageous in terms of convenience.

The method for measuring the expression level of ApoA4 protein is not particularly limited as long as it is a method that can specifically detect and measure ApoA4 protein, and methods ordinarily employed for measuring can be applied. Such a method can include, but is not limited to, for example, a method for measuring ApoA4 protein or a fragment thereof such as a method that employs an antibody that binds to ApoA4 protein, ion exchange chromatography, mass spectrometry, LC/MS, and LC/MS/MS having a mass spectrometer tandemly connected to LC/MS.

In the present invention, the device employed for the detection of ApoA4 protein is not particularly limited, and can be suitably selected depending on the method for measuring ApoA4 protein. Specifically, this can include, for example, an HPLC instrument, a mass spectrometry instrument (mass spectrometry), a LC/MS instrument having liquid chromatography and mass spectrometry instruments connected, LC/MS/MS instrument having a mass spectrometer tandemly connected to an LC/MS instrument, an electrophoresis instrument (such as a capillary electrophoresis device), an automatic or semi-automatic enzyme immunoassay instrument, a cell washer, an automatic or semi-automatic chemiluminescent immunoassay instrument, a luminescence measuring device, an automatic or semi-automatic electrochemiluminescent immunoassay instrument, an optical measuring device, a plate reader, a CCD camera, an automatic or semi-automatic fluorescent immunoassay instrument, a fluorescence measuring device, an automatic or semi-automatic radioimmunoassay instrument, a liquid scintillation counter, a Coulter counter, a surface plasmon measuring device, a blotting device, a densitometer, and the like.

In one embodiment of the present invention, it is preferred to use a method that employs an antibody that binds to ApoA4 protein (also referred to herein as an "anti-ApoA4 antibody"), for example an immunoassay that employs an anti-ApoA4 antibody for measuring the expression level of ApoA4 protein. The anti-ApoA4 antibody is not particularly limited as long as it is an antibody that can recognize and bind ApoA4.

The anti-ApoA4 antibody including both monoclonal and polyclonal antibodies can be created according to well-known methods. A monoclonal antibody can be obtained by for example isolating an antibody-producing cell from a non-human mammal immunized with ApoA4 or an ApoA4 fragment, fusing this with a myeloma cell etc. to create a hybridoma, and purifying the antibody produced by this hybridoma. Moreover, a polyclonal antibody can be obtained from the serum of an animal immunized with an ApoA4 protein or an ApoA4 fragment. An ApoA4 fragment is a partial peptide of the ApoA4 protein, and an anti-ApoA4 fragment antibody recognizes the ApoA4 protein. Moreover, examples of immunogens include, but are not limited to, the ApoA4 protein or an ApoA4 fragment of for example primates such as human or monkey, rodents such as rat or mouse.

Note that an "antibody" herein refers to a full length immunoglobulin molecule or an immunologically active fragment of an immunoglobulin molecule such as an antibody fragment that occurs in nature or is produced by genetic recombination technology. These antibodies can be created with conventional technology, and may be a polyclonal antibody or a monoclonal antibody. Antibody fragments include F(ab')₂, F(ab)₂, Fab', Fab, Fv, scFv, and the like.

The anti-ApoA4 antibody used in the present invention is preferably an antibody that specifically binds to the ApoA4 protein. "Specific binding" refers to an antibody that binds to the ApoA4 protein at a higher binding affinity compared to non-specific interaction. In one embodiment of the present invention, "specific binding" may be shown by for example an antibody that has a Kd of at least about 10⁻⁴ M, or at least about 10⁻⁵ M, or at least about 10⁻⁶ M, or at least about 10⁻⁷ M, or at least about 10⁻⁸ M, or at least about 10⁻⁹ M, or at least about 10⁻¹⁰ M, or at least about 10⁻¹¹ M, or at least about 10⁻¹² M, or more against the ApoA4 protein. In another embodiment, "specific binding" means binding to ApoA4 without substantially binding to other polypeptides different from the ApoA4 polypeptide.

An immunoassay employs a detectably labeled anti-ApoA4 antibody, or an antibody (secondary antibody) against a detectably labeled anti-ApoA4 antibody. Depending on the labeling method of the antibody, it is classified into enzyme immunoassay (EIA or ELISA), radioimmunoassay (RIA), fluorescence immunoassay (FIA), fluorescence polarization immunoassay (FPIA), chemiluminescence immunoassay (CLIA), electrochemiluminescence immunoassay (ECLIA), and the like, all of which can be employed in the method of the present invention.

ELISA method can employ an antibody labeled with an enzyme such as peroxidase and alkaline phosphatase, RIA method with a radioactive material such as ¹²⁵I, ¹³¹I, ³⁵S, and ³H, FPIA method with a fluorescent substance such as fluorescein isothiocyanate, rhodamine, dansyl chloride, phycoerythrin, tetramethylrhodamine isothiocyanate, and near-infrared fluorescent material, and CLIA method can employ an enzyme such as luciferase and β galactosidase and an antibody labeled with a luminescent substrate that changes into a luminescent substance with the respective enzyme or a luminescent substance such as luciferin and aequorin. Furthermore, an antibody labeled with a nanoparticle such as a gold colloid and a quantum dot can also be detected.

Moreover, in an immunoassay, an anti-ApoA4 antibody can be labeled with biotin, and allowed to bind to avidin or streptavidin labeled with for example an enzyme in order to detect and measure the ApoA4.

Among immunoassays, ELISA method employing enzyme-labeling is preferred in that it allows convenient and rapid measurement of the target.

In ELISA method, sandwich method can for example be used. An anti-ApoA4 antibody is fixed onto a solid phase support, a suitably treated biological sample is added and allowed to react, and further, an anti-ApoA4 antibody that recognizes another epitope labeled with an enzyme is added and allowed to react.

After washing, the expression level of ApoA4 protein can be determined by allowing reaction with the enzyme substrate, and then subjecting to color development and measuring the absorbance. Moreover, after allowing the anti-ApoA4 antibody fixed onto the solid phase support with the ApoA4 protein in the biological sample to react, unlabeled anti-ApoA4 antibody (primary antibody) may be added, and an antibody (secondary antibody) against this unlabeled antibody may be enzyme-labeled and further added.

For the enzyme substrate, 3,3'-diaminobenzidine (DAB), 3,3',5,5'-tetramethylbenzidine (TMB), o-phenylenediamine (OPD), and the like can be employed when the enzyme is a peroxidase, and p-nitrophenyl phosphate (NPP) and the like can be employed when it is alkaline phosphatase.

Moreover, in the above immunoassay, the aggregation method is also preferred as a method for conveniently detecting a trace amount of protein. An example of the aggregation method includes the latex aggregation method where a latex particle is bound to the antibody.

When a latex particle is bound to the anti-ApoA4 antibody and mixed with a biological sample, the antibody-bound latex particles aggregate if the ApoA4 protein is present. Near-infrared light is then irradiated onto the sample, the aggregation mass is quantified by measurement of absorbance (turbidimetry) or measurement of scattered light (nephelometry), and the antigen concentration can be determined.

The measurement of the expression level of ApoA4 gene is not particularly limited as long as it is a method that can quantitatively detect ApoA4 mRNA, and for example, a nucleic acid amplification method such as PCR method, RT-PCR method, and real-time PCR method employing a primer specific to ApoA4 mRNA can be employed. For example, real-time PCR be carried out specifically as follows. In other words, total RNA is acquired from tissue according to conventional means, and cDNA is created with a reverse transcription enzyme and a suitable primer. Based on the created cDNA, the ApoA4 gene can be specifically amplified by employing a primer specific to the ApoA4 gene and a DNA polymerase. The amplification curve of the ApoA4 gene can be monitored with a particular instrument (such as PRISM 7700 Sequence Detector 7900HT Sequence Detection System or ViiA™ 7 Real-Time PCR System (from Applied Biosystems)) by incorporating a suitable fluorescent dye upon amplification. By correcting ApoA4 by an internal standard gene (such as Hprt), the amount of ApoA4 mRNA in the tissue can be quantitatively measured.

In another aspect, the present invention relates to a kit for evaluating the pharmacodynamic action of HGF. The kit according to the present invention comprises a means for measuring the ApoA4 level, and in one embodiment, such a means is an anti-ApoA4 antibody, an ApoA4 aptamer, an ApoA4 protein, or a fragment thereof, or the same labeled with an isotope, or a primer or a probe consisting of a partial sequence complementary to the ApoA4 gene sequence or the same labeled with a dye. In a preferred embodiment of the kit of the present invention, the anti-ApoA4 antibody is an antibody that specifically binds to ApoA4, and the ApoA4 aptamer is an aptamer that specifically binds to the ApoA4 protein.

The kit of the present invention may further comprise reagents and devices necessary for measuring the amount of ApoA4 protein in the biological sample by an immunoassay that utilizes the antigen antibody reaction between the ApoA4 protein and the anti-ApoA4 antibody.

In one embodiment, the kit of the present invention is for measuring the amount of ApoA4 protein by sandwich ELISA method, and comprises a microtiter plate; an anti-ApoA4 antibody for capture; an anti-ApoA4 antibody labeled with alkaline phosphatase or peroxidase; and an alkaline phosphatase substrate (such as NPP) or a peroxidase substrate (such as DAB, TMB, and microtiter plate OPD). The capture antibody and the labeled antibody recognize different epitopes. In such a kit, first, the capture antibody is fixed onto a microtiter plate, a suitably treated and diluted biological sample is added thereto and incubated, and the sample is removed and washed. Next, the labeled antibody is added and incubated, the substrate is added, and this is subjected to color development. By measuring the color development with a microtiter plate reader etc., the amount of ApoA4 protein can be determined.

In another embodiment, the kit of the present invention is for measuring the amount of ApoA4 protein by sandwich ELISA method that uses a secondary antibody, and comprises a microtiter plate; an anti-ApoA4 antibody for capture; an anti-ApoA4 antibody as the primary antibody; an antibody against the primary anti-ApoA4 antibody labeled with alkaline phosphatase or peroxidase as the secondary antibody; and an alkaline phosphatase (such as NPP) or a peroxidase substrate (such as DAB, TMB, and OPD). The capture antibody and the primary antibody recognize different epitopes. In such a kit, first, the capture antibody is fixed onto a microtiter plate, a suitably treated and diluted biological sample is added thereto and incubated, and the sample is removed and washed. Subsequently, the primary antibody is added and incubated and washed, the enzyme-labeled secondary antibody is further added and incubated, and then the substrate is added and subjected to color development. By measuring the color development with a microtiter plate reader etc., the amount of ApoA4 protein can be determined. By employing a secondary antibody, the reaction is amplified and detection sensitivity can be improved.

It is also preferred that the kit of the present invention further comprises the necessary buffer, enzyme reaction quenching solution, a microplate reader, product instruction, and the like.

The labeled antibody is not limited to an enzyme-labeled antibody, and may be an antibody labeled with a radioactive material (such as ¹²⁵I, ¹³¹I, ³⁵S, and ³H), a fluorescent substance (such as fluorescein isothiocyanate, rhodamine, dansyl chloride, phycoerythrin, tetramethylrhodamine isothiocyanate, and near-infrared fluorescent material), a luminescent substance (such as luciferase, luciferin, and aequorin), a nanoparticle (gold colloid and quantum dot), and the like. Moreover, a biotinylated antibody may also be employed as the labeled antibody, and labeled avidin or streptavidin may be added to the kit.

The kit of the present invention may further comprise reagents and devices necessary for measuring the amount of ApoA4 in a biological sample in an assay that utilizes the reaction between ApoA4 and ApoA4 aptamer.

Note that the terms used herein are to be employed to describe particular embodiments and do not intend to limit the invention.

Moreover, the term "comprising" as used herein, unless the content clearly indicates to be understood otherwise, intends the presence of the described items (such as components, steps, elements, and numbers), and does not exclude the presence of other items (such as components, steps, elements, and numbers).

Unless otherwise defined, all terms used herein (including technical and scientific terms) have the same meanings as those broadly recognized by those skilled in the art of the technology to which the present invention belongs. The terms used herein, unless explicitly defined otherwise, are to be construed as having meanings consistent with the meanings herein and in related technical fields, and shall not be construed as having idealized or excessively formal meanings.

Terms such as first and second are sometimes employed to express various elements, and it should be recognized that these elements are not to be limited by these terms. These terms are employed solely for the purpose of discriminating one element from another, and it is for example possible to describe a first element as a second element, and similarly, to describe a second element as a first element without departing from the scope of the present invention.

The present invention will now be more specifically described by Examples. However, the present invention can be embodied by various embodiments, and shall not be construed as being limited to the Examples described herein.

### Examples

### [Example 1] Gene expression analysis employing hepatic tissue of healthy mice administered rh-HGF

### Object:

Using liver which is a target organ to which rh-HGF brings its biological activity as a material, identification of molecules affected by rh-HGFand identification of a PD marker among the moleculeswere attempted. Microarray and real-time PCR method were employed for molecule identification.

### Experiment:

### Administration of rh-HGF and harvest

Using hepatocyte growth factor activator recombinantly expressed in Chinese hamster ovary (CHO) cells, human HGF recombinantly expressed in CHO cells were activated to obtain rh-HGF. BALB/cAnNCrlCrlj male 6 weeks-old mice (CHARLES RIVER JAPAN) were intravenously administered with Dulbecco's Phosphate buffered saline (PBS) (Wako, #045-29795) or rh-HGF (1.5 mg/kg). Immediately after PBS administration and 8 and 24 hours after administration of rh-HGF, blood was collected from the abdominal inferior vena cava under anesthesia by 3% isoflurane inhalation, and mice were euthanized by cervical dislocation method. After euthanization, hepatic tissue was collected and immersed in RNA later (Ambion, #AM7021). The Table below shows the test groups employed in this experiment.

**[Table 1]**

| <Group configuration> | | | |
|---|---|---|---|
| Group | Administration | Timing of harvest after administration | Number of mouse individuals |
| 1 | PBS | 0 immediately after | 3 |
| 2 | rh-HGF 1.5 mg/kg | 8 hr | 3 |
| 3 | rh-HGF 1.5 mg/kg | 24 hr | 3 |

### Extraction of total RNA derived from liver

From the harvested liver, total RNA was purified with TRIZOL Reagent (Invitrogen, #15596-018), RNeasy Mini Kit (QIAGEN, #74104), and RNase-Free DNase set (QIAGEN, #79254). RNA concentration was measured by absorbance, and then quality check was carried out with RNA 6000 Nano Assay Kit (Agilent Technologies, #5067-1511) using 2100 Bioanalyzer (Agilent Technologies).

### DNA microarray analysis

The DNA microarray used was SurePrint G3 mouse GE 8 X 60K (Agilent Technologies, #G4852A). Among the data obtained, genes which had a signal value of 100 or more in all three groups were targeted, and those having two-folds or more increase in the signal value in Group 2 or Group 3 compared to Group 1 were temporarily extracted. Among this, as molecules that may appear to possibly exist as soluble proteins, those having information of "Extracellular" or "Plasma membrane" in Gene Ontology annotation were extracted. In this way, molecules of which expression was strongly induced by rh-HGF administration were selected as PD marker candidates.

### Result 1:

ApoA4 molecules were extracted by the above molecule selection method (Table 2). ApoA4 molecules are molecules that exist in the blood as apoproteins that configure apolipoproteins. ApoA4 molecules had low expression in mice that were administered PBS only (Group 1), and enhancement of expression was seen with rh-HGF administration.

**[Table 2]**

| | | | | Change in expression (vs 0 hours) | | | |
|---|---|---|---|---|---|---|---|
| Gene code (probe ID) | Signal value (average) | | | 8 hours | | 24 hours | |
| | 0 hours | 8 hours | 24 hours | Ratio | P value | Ratio | P value |
| ApoA4 (A_51_ P327491) | 2436 | 15738 | 13453 | 6.46 | 0.0002 | 5.52 | 0.0016 |

| | | | | | | | |
|---|---|---|---|---|---|---|---|
| P value: Welch's test | | | | | | | |

### Confirmation of induction of ApoA4 mRNA expression by real-time PCR

The same RNA as the sample employed for DNA microarray was reverse transcripted using High-Capacity cDNA Reverse Transcription Kit with RNase Inhibitor (Applied Biosystems, #4374966) to create cDNA. With the cDNA obtained as the template, the amount of ApoA4 mRNA expressed was examined by real-time PCR method. Hprt mRNA was used as the internal control for examination. For PCR reaction, PCR amplification reaction was carried out with TaqMan Fast Advanced Master Mix (Applied Biosystems, #4444557) and TaqMan Assay (ApoA4: Mm00431814_m1, Hprt: Mm03024075_m1, Applied Biosystems) and using ViiA7 Real-time PCR System (Applied Biosystems). For the amplification reaction, incubation at 95°C for 20 seconds, and then reaction at 95°C for 1 second and 60°C for 20 seconds were repeated for 40 cycles. Correction between samples was carried out by standardizing the expression value of ApoA4 mRNA for each sample with the expression value of Hprt mRNA.

### Statistical analysis:

With the results of ApoA4 mRNA quantification, Dunnett's multiple comparison test was carried out between the control and the rh-HGF administration groups (Figure 1).

### Result 2:

With ApoA4 obtained as a result of DNA microarray, the mRNA thereof was quantified and determined with real-time PCR method. As a result of ApoA4 mRNA quantification with the mRNA of a housekeeping gene Hprt as the internal control, it was confirmed that its behavior was correlated with the fluctuation of the signal value seen in the DNA microarray, and that expression was strongly enhanced by rh-HGF administration (Figure 1).

### [Example 2] ApoA4 induction by rh-HGF administration and cancelling effect by c-Met inhibitor

### Object:

The biological activity of rh-HGF is achieved via its receptor, c-Met. In other words, the validity that ApoA4 is a PD marker of rh-HGF can be guaranteed by revealing that the induced expression is dependent on c-Met. Accordingly, using healthy mice that were administered a specific inhibitor of c-Met, the cancelling effect of induction of ApoA4 expression by rh-HGF administration was investigated. Investigation targets were mRNA derived from liver and protein in the serum.

### Experiment:

### Administration of c-Met inhibitor and experiment groups

To BALB/cAnNCrlCrlj male 6 weeks-old mice (CHARLES RIVER JAPAN), c-Met inhibitor PF-04217903 (50 mg/kg) (Selleck Chemicals #S1094, lot. 05) or vehicle (5% DMSO, 10% Tween80, 6.7 mmol/L aqueous hydrochloric acid solution) was orally administered at a dose of 10 mL/kg, and after 1 hour, rh-HGF (0.5, 1.5, 5.0, or 15 mg/kg) or PBS was intravenously administered. Twenty-four hours after administration of rh-HGF or PBS, blood was collected from the abdominal inferior vena cava under anesthesia by 3% isoflurane inhalation, and mice were euthanized by cervical dislocation method. After euthanization, hepatic tissue was collected and immersed in RNA later (Ambion, #AM7021). Serum was collected from the blood collected. The Table below shows the test groups employed in this experiment.

**[Table 3]**

| <Group configuration> | | | |
|---|---|---|---|
| Group | c-Met inhibitor (mg/kg) | rh-HGF (mg/kg) | Number of mouse individuals |
| 1 | 0 | 0 | 6 |
| 2 | 0 | 0.5 | 6 |
| 3 | 0 | 1.5 | 6 |
| 4 | 0 | 5 | 6 |
| 5 | 0 | 15 | 6 |
| 6 | 50 | 0 | 6 |
| 7 | 50 | 0.5 | 6 |
| 8 | 50 | 1.5 | 6 |
| 9 | 50 | 5 | 6 |
| 10 | 50 | 15 | 6 |

### Extraction of total RNA derived from liver

From the harvested liver, total RNA was purified with TRIZOL Reagent (Invitrogen, #15596-018), RNeasy Mini Kit (QIAGEN, #74104), and RNase-Free DNase set (QIAGEN, #79254), and RNA concentration was measured by absorbance.

### Quantification of Apoa4 mRNA by real-time PCR method

Quantification of ApoA4 mRNA by real-time PCR method was carried out according to Example 1.

### Statistical analysis:

With the results of ApoA4 mRNA quantification, Dunnett's multiple comparison test was carried out between the control and the rh-HGF administration groups. Further, with the rh-HGF treatment group where ApoA4 mRNA increased statistically significantly, unpaired t-test was carried out between the vehicle and the c-Met inhibitor treatment groups (Figure 2).

### Result 1:

It was reconfirmed that ApoA4 mRNA expression was significantly induced/enhanced in the liver by rh-HGF administration. Further, since the induction was significantly inhibited by administering a c-Met inhibitor, it could be confirmed that enhancement of ApoA4 mRNA expression accompanying rh-HGF administration was dependent on c-Met (Figure 2).

### Quantification of serum ApoA4 protein

Using the above serum, ApoA4 protein was quantified by Western blot method. To the serum diluted 200-folds with PBS (-) (Wako), 1/4 volume of the electrophoresis buffer solution (313 mM Tris-HCl (pH 6.8), 10% SDS, 30% Glycerol, 0.2 mg/mL Bromo-phenol blue, and 25% 2-Mercaptoethnol) was added (the final dilution of the serum will be 250-folds), and this was subjected to heat treatment at 95°C for 3 minutes. Of this, 12.5 µL was subjected to SDS-PAGE, and blotted onto a nitrocellulose filter (Schleicher & Schuell, BA85). The filter was soaked in the blocking solution (50 mM Tris-HCl (pH 7.5), 150 mM NaCl, 5% skim-milk, 0.01% Tween20, and 0.1% NaN3), shaken at room temperature for 1 hour, then the blocking solution was discarded, the filter was soaked in a reaction solution (50 mM Tris-HCl (pH 7.5), 150 mM NaCl, 5% skim-milk, 4% PEG6000, 0.2% EDTA/3Na, 0.01% Tween20, and 0.2% Proclin150) supplemented with goat anti-mouse Apolipoprotein A-IV antibody (GeneTex, #GTX88533) to 0.42 µg/mL concentration, and shaken at room temperature for 2 hours. After washing three times with the wash solution (50 mM Tris-HCl (pH 7.5), 150 mM NaCl, and 0.01% Tween20), the filter was soaked in the above reaction solution supplemented with the secondary antibody which is HRP-labeled rabbit anti-goat IgG (H+L) (Invitrogen) to 1/2000 concentration, and shaken at room temperature for 1 hour. After washing three times with the above wash solution, chemiluminescence solution SuperSignal West Dura Extended Duration Substrate (Thermo Scientific) was added to allow luminescence, and ApoA4 protein was detected with LAS-4000 (FUJIFILM).

### Statistical analysis:

For the results of serum ApoA4 protein quantification, Dunnett's multiple comparison test was carried out between the control and the rh-HGF administration groups. Further, with the rh-HGF treatment group where ApoA4 protein increased statistically significantly, unpaired t-test was carried out between the vehicle treatment group and the c-Met inhibitor treatment group (Figure 3).

### Result 2:

The induction of ApoA4 protein expression by rh-HGF administration could be detected as protein in the serum. Further, since the induction was inhibited by administering a c-Met inhibitor, it could be confirmed that enhancement of ApoA4 protein expression accompanying rh-HGF administration was dependent on c-Met (Figure 3).

### [Example 3] Confirmation of enhancement effect of ApoA4 protein production by h-HGF administration during experimental hepatopathy

### Object:

From previous results, it was thought that mouse ApoA4 protein was a PD marker of HGF that can be measured in blood. ApoA4 protein behaved as a PD marker of HGF in the liver mRNA and blood of healthy mice, then another test with experimental hepatopathy mice (Jo2 liver failure model) was carried out to confirm whether it also behaves as a PD marker of HGF during hepatopathy. The Jo2 liver failure model is a hepatopathy model with high severity attributed to direct evoking of hepatocyte apoptosis by administration of anti-Fas monoclonal antibody (Anti-Fas MAb).

### Experiment:

### Fabrication of Jo2 liver failure model

To BALB/cAnNCrlCrlj male 6 weeks-old mice (CHARLES RIVER JAPAN), 0.23 mg/kg of anti-Fas monoclonal antibody (clone name: Jo2, BD Pharmingen, #554254, lot#3269634) was administered once into the tail vein to create an acute liver failure model. To this model, rh-HGF dissolved in PBS was administered into the tail vein at 0.15, 1.5, or 15 mg/kg 1 hour before administration of anti-Fas monoclonal antibody. For the control group (Control), PBS was administered into the tail vein.

### Evaluation of severity of hepatopathy

Five hours after the administration of anti-Fas monoclonal antibody, mice were retained under anesthesia by 3% isoflurane inhalation, blood was collected from the vein located behind the jaw bone, and Prothrombin time (PT) was measured with CoaguChek XS (Roche-diagnostics, #ES350009) and PT Test strips (Roche-diagnostics, #ES350054). After measuring the Prothrombin time, blood was collected from the abdominal inferior vena cava, and the mice were then euthanized by cervical dislocation method. The measurement of Aspartate Aminotransferase (AST) and Alanine transaminase (ALT) in the serum was each performed with L-type Wako AST·J2 (Wako) kit and L-type Wako ALT·J2 (Wako) kit and an automatic analysis device (Hitachi 7180 Automatic Analyzer, Hitachi High-Technologies).

### Quantification of serum ApoA4 protein

Quantification of serum ApoA4 protein was carried out according to Example 2.

### Statistical analysis:

With the measurement results of PT, AST, ALT, and serum ApoA4 protein, Dunnett's multiple comparison test was carried out between the control and the rh-HGF administration groups (Figure 4).

### Result:

In Jo2 liver failure mice, although significant elevation of AST and ALT as well as extension of PT were recognized in the control, these were strongly inhibited by rh-HGF administration. At this time, blood ApoA4 protein value was elevated by administration of rh-HGF, and showed significant elevation at 1.5, 5, or 15 mg/kg. Accordingly, it was found that elevation of ApoA4 protein was seen that reflects the effect of preventing hepatopathy by HGF (Figure 4) .

### [Example 4] Confirmation of ApoA4 protein as PD marker with human primary culture hepatocytes

### Object:

It was confirmed through Examples with mice that blood ApoA4 protein is a PD marker of HGF. To confirm whether ApoA4 protein may in fact also be a PD marker of HGF in human, a test with human primary culture hepatocytes was carried out.

### Experiment:

### Human primary culture hepatocytes and treatment by rh-HGF

Human primary culture hepatocytes seeded in a 24-well plate (3.8 X 10⁵ cells/well, Biopredic International, #HEP220-MW24) were cultured for three days in Incubation Medium (product name: Biopredic International, #MIL214), then c-Met inhibitor PF-04217903 (final concentration 100 nmol/L) (Selleck Chemicals, #S1094, lot. 05) or vehicle (DMSO) was added, and after 1 hour, rh-HGF (final concentration 100, 300, or 1000 ng/mL) or (Incubation Medium) for the control group (Control) was added.

### Quantification of human ApoA4 mRNA

From hepatocytes 24 hours after addition of rh-HGF, total RNA was purified with RNeasy Mini Kit (QIAGEN, #74104) and RNase-Free DNase set (QIAGEN, #79254), and RNA concentration was measured by absorbance. The purified total RNA was reverse transcripted using High-Capacity cDNA Reverse Transcription Kit with RNase Inhibitor (Applied Biosystems, #4374966) to create cDNA. With the cDNA obtained as the template, the amount of ApoA4 mRNA expressed was examined by real-time PCR method. 18S rRNA was used as the internal control for examination. For PCR reaction, PCR amplification reaction was carried out with TaqMan Fast Advanced Master Mix (Applied Biosystems, #4444557) and TaqMan Assay (ApoA4: Hs00166636_m1, 18S rRNA: Hs99999901_s1, Applied Biosystems) and using ViiA7 Real-time PCR System (Applied Biosystems). For the amplification reaction, incubation at 95°C for 20 seconds, and then reaction at 95°C for 1 second and 60°C for 20 seconds were repeated for 40 cycles. Correction between samples was carried out by standardizing the expression value of ApoA4 mRNA for each sample with the expression value of 18S rRNA.

### Statistical analysis:

With the results of ApoA4 mRNA quantification, Dunnett's multiple comparison test was carried out between the control and the rh-HGF administration groups. With the rh-HGF treatment group where ApoA4 mRNA increased statistically significantly, unpaired t-test was carried out between the vehicle treatment group and the c-Met inhibitor treatment group (Figure 5).

### Result:

By exposing human primary culture hepatocytes to rh-HGF, human ApoA4 mRNA expression was strongly enhanced. Moreover, since this enhancement effect was inhibited by the specific c-Met inhibitor, it was strongly suggested that ApoA4 is also a PD marker that reflects the biological activity of rh-HGF via c-Met in human hepatocytes. Accordingly, it was suggested that ApoA4 is also a PD marker of rh-HGF in human (Figure 5).

### Quantification of human ApoA4 protein

Using hepatocyte culture supernatant (the same culture supernatant as that of cells for preparation of mRNA) 24 hours after addition of rh-HGF, ApoA4 protein was quantified by Western blot method. To the culture supernatant diluted 4-folds with PBS (Wako), 1/4 volume of the electrophoresis buffer solution (313 mM Tris-HCl (pH 6.8), 10% SDS, 30% Glycerol, 0.2 mg/mL Bromo-phenol blue, 25% 2-Mercuptoethnol) was added (the final dilution of culture supernatant was 20-folds), and this was subjected to heat treatment at 95°C for 3 minutes. Of this, 10 µL was subjected to SDS-PAGE, and blotted onto a nitrocellulose filter (Schleicher & Schuell, BA85). The filter was soaked in the blocking solution (50 mM Tris-HCl (pH 7.5), 150 mM NaCl, 5% skim-milk, 0.01% Tween20, and 0.1% NaN3), shaken at room temperature for 1 hour, then the blocking solution was discarded, the filter was soaked in a reaction solution (50 mM Tris-HCl (pH 7.5), 150 mM NaCl, 5% skim-milk, 4% PEG6000, 0.2% EDTA/3Na, 0.01% Tween20, and 0.2% Proclin150) supplemented with rabbit anti-human Apolipoprotein A4 antibody (Proteintech, 17996-1-AP) to 0.067 µg/mL concentration, and shaken at room temperature for 2 hours. After washing three times with the wash solution (50 mM Tris-HCl (pH 7.5), 150 mM NaCl, and 0.01% Tween20), the filter was soaked in the above reaction solution supplemented with the secondary antibody which is HRP-labeled goat anti-rabbit IgG (H+L) (Jackson) to 1/2000 concentration, and shaken at room temperature for 1 hour. After washing three times with the above wash solution, chemiluminescence solution SuperSignal West Dura Extended Duration Substrate (Thermo Scientific) was added to allow luminescence, and ApoA4 protein was detected with LAS-4000 (FUJIFILM).

### Statistical analysis:

With the quantification results of ApoA4 protein, Dunnett's multiple comparison test was carried out between the control and the rh-HGF administration groups. Then, with the rh-HGF treatment group where ApoA4 protein increased statistically significantly, unpaired t-test was carried out between the vehicle treatment group and the c-Met inhibitor treatment group (Figure 6).

### Result 2:

By exposing human primary culture hepatocytes to rh-HGF, extracellular release of human ApoA4 protein was strongly enhanced. Moreover, since this enhancement effect was inhibited by the specific c-Met inhibitor, it was strongly suggested that ApoA4 protein is also a PD marker that reflects the biological activity of rh-HGF via c-Met in human hepatocytes. Accordingly, it was suggested that ApoA4 protein is also a PD marker of rh-HGF in human (Figure 6).

## Claims

1. A method for evaluating the pharmacodynamic action of hepatocyte growth factor (HGF), **characterized in that** it comprises a step of measuring the ApoA4 level in a biological sample collected from a test subject who was administered HGF.

2. The evaluation method according to claim 1, **characterized in that** it further comprises a step of comparing the ApoA4 level in a biological sample collected from a test subject who was administered HGF with the ApoA4 level in said biological sample collected from a test subject who was not administered HGF or who has a disappeared influence from the administration of HGF.

3. The evaluation method according to claim 2, **characterized in that** it further comprises a step of determining that said administered HGF is administered in an amount that yields pharmacodynamic action when the ApoA4 level of a test subject who was administered HGF is elevated compared to the ApoA4 level of a test subject who was not administered HGF or who has a disappeared influence from the administration of HGF.

4. The evaluation method according to any one of claims 1 to 3, **characterized in that** said "ApoA4 level" is the expression level of ApoA4 protein.

5. The evaluation method according to claim 4, **characterized in that** the expression level of ApoA4 protein in the biologically derived sample is measured by an immunoassay.

6. The evaluation method according to any one of claims 1 to 5, **characterized in that** said biologically derived sample is a sample derived from serum or blood plasma.

7. The evaluation method according to any one of claims 1 to 3, **characterized in that** said "ApoA4 level" is the expression level of ApoA4 gene.

8. The evaluation method according to claim 7, **characterized in that** the expression level of ApoA4 gene in the biologically derived sample is measured by real-time PCR.

9. The evaluation method according to any one of claims 1 to 3, 7, or 8, **characterized in that** said biologically derived sample is a hepatocyte or hepatic tissue.

10. The evaluation method according to any one of claims 1 to 9, **characterized in that** said test subject is a healthy individual.

11. The evaluation method according to any one of claims 1 to 9, **characterized in that** said test subject is a test subject having liver disease.

12. A kit for evaluating the pharmacodynamic action of HGF, **characterized in that** it comprises an anti-ApoA4 antibody.
